# EUROPEAN PATENT APPLICATION

(11) **EP 2 108 403 A1**
(43) Date of publication of application: **14.10.2009**
(21) Application number: 09005164.0
(22) Date of filing: 08.04.2009
(51) Int. Cl.: A61N 7/02

(54) **Improved ultrasound device for treating fat tissue**

(30) Priority: 09.04.2008 IT MI20080628
(71) Applicant: Novavision Group S.r.l, 20123 Milano (IT)
(72) Inventor: Crapelli, Danilo, 20123 Milano (IT)
(74) Representative: Cicogna, Franco

(57) **Abstract**

An improved ultrasound device, specifically designed for treating fat or adipose tissue, is **characterized in that** said device comprises a control, apparatus, which, being connected to a CPU with a dedicated software, prevents the temperature of the instrument handle and related parts connected thereto, from increasing, to prevent the skin surface from being burnt, while further preventing the emitter, during its operation, from being held in a stationary condition on a single point, thereby preventing the tissue from being necrotized.

## Description

### BACKGROUND OF THE INVENTION

The present invention relates to an improved ultrasound device, which has been specifically designed for treating fat or adipose tissue.

As is known, cavitation is a phenomenon consisting of a formation of steam regions inside a liquid, which steam regions are subjected to implosion.

This occurs because of a localized pressure lowering to a value less than the vapor tension of the liquid, which is subjected to a phase changing to a gas, thereby forming vapor or steam holding cavities.

A vapor containing cavity, in particular, will resist as far as it exits the low hydrostatic pressure region: as it returns to a rest-fluid region, however, the vapor pressure is not sufficient to overcome the hydrostatic pressure, and the cavitation "bubble" will immediately implode while freeing implosion energy.

In an ultrasound cavitation, ultrasound vibrations will generate through the liquid waves, which, alternating a compression step to a following decompression step, will generate a very high turbulence with a consequent cavitation bubble formation, said cavitation bubbles then imploding by providing a very high pressure.

The greater is the compression and decompression action, the larger will be the generated energy.

The ultrasound cavitation phenomenon is exploited, for example, in the aesthetic medical field, for reducing or re-patterning the fat or adipose tissue.

The fat properties of an adipose tissue are measured, as is also known, by using morpho-functional adipocite units.

Said adipocites are suitable to hold therein variable amounts of fat or adipose materials, and frequently form fat masses, divided into lobules by connective "sepiments" or dividing elements, therein the blood vessels are located.

In particular, the above cavitation phenomenon also allows to controllably reduce the excess fat material and cellulite slight blemishes, through a non-invasive, short and safe treatment.

In fact, the impact wave caused by an implosion of the "bubble" in the adipose tissue, facilitates a draining of the liquid materials and a release of the fat acids by the cells, with a consequent visible reduction of the excess volume.

The above phenomenon has both a mechanical and a thermal effect.

The mechanical action is due to the movement of the particles of tissues traversed by ultrasounds, whereas the thermal effect is a direct consequence of the mechanical effect, since the heat will be generated by the vibration, impact and friction of the cellular and intracellular structures constituting the tissues the ultrasounds pass therethrough.

The above mentioned temperature increase is developed both on a surface and a depth level, and is a very quick action.

The mechanical effect generated by ultrasounds and consequent implosion of the cavitational "bubbles" could release an amount of energy sufficient to cause a thermal necrosis of the local cellular population.

A possible heating of the ultrasound instrument handle, caused by an extended and continuous use, on the other hand, could cause a skin surface burning.

### SUMMARY OF THE INVENTION

Accordingly, the aim of the present invention is to provide such an improved ultrasound device suitable to solve the above mentioned problems affecting the prior art.

Within the scope of the above mentioned aim, a main object of the invention is to provide such an improved ultrasound device designed for preventing any increase of the device handle and related parts connected thereto, to prevent the surface of the skin to be burnt, and which, moreover, cannot be held at a stationary place, on a single point, to further prevent the tissue from being necrotized.

Another object of the present invention is to provide such an improved ultrasound device which, owing to its specifically designed constructional features, is very reliable and safe in operation.

Yet another object of the present invention is to provide such an improved ultrasound device which can be easily made starting from easily available elements and materials and which, moreover, is very competitive from a mere economic standpoint.

The above mentioned aim and objects, as well as yet other objects, which will become more apparent hereinafter, are achieved by an improved ultrasound device, specifically designed for treating fat or adipose tissue, characterized in that said device comprises a control apparatus which, being connected to a CPU with a dedicated software, prevents a temperature of a device handle and parts coupled thereto, from increasing so as to prevent a skin surface from being burnt, while preventing the ultrasound emitter from being held stationary at a single place, to prevent a tissue from being necrotized.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:

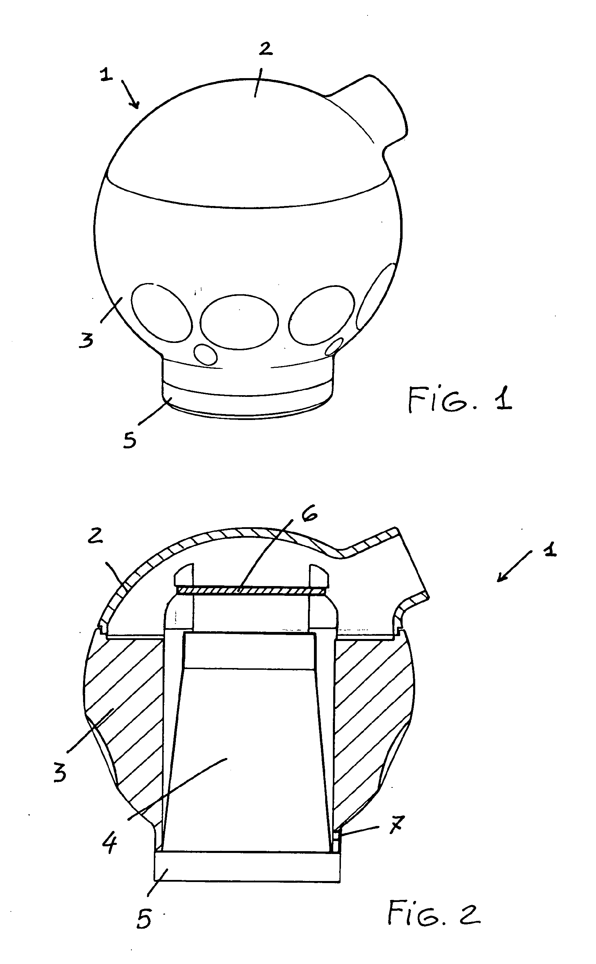

Figure 1 is a perspective view of the improved ultrasound device according to the present invention;
Figure 2 is a cross-sectional view of the improved ultrasound device according to the invention;
Figure 3 is a block diagram showing a control apparatus designed for controlling the device according to the invention;
Figure 4 is a further block diagram of an ultrasound emitter included in the inventive device; and

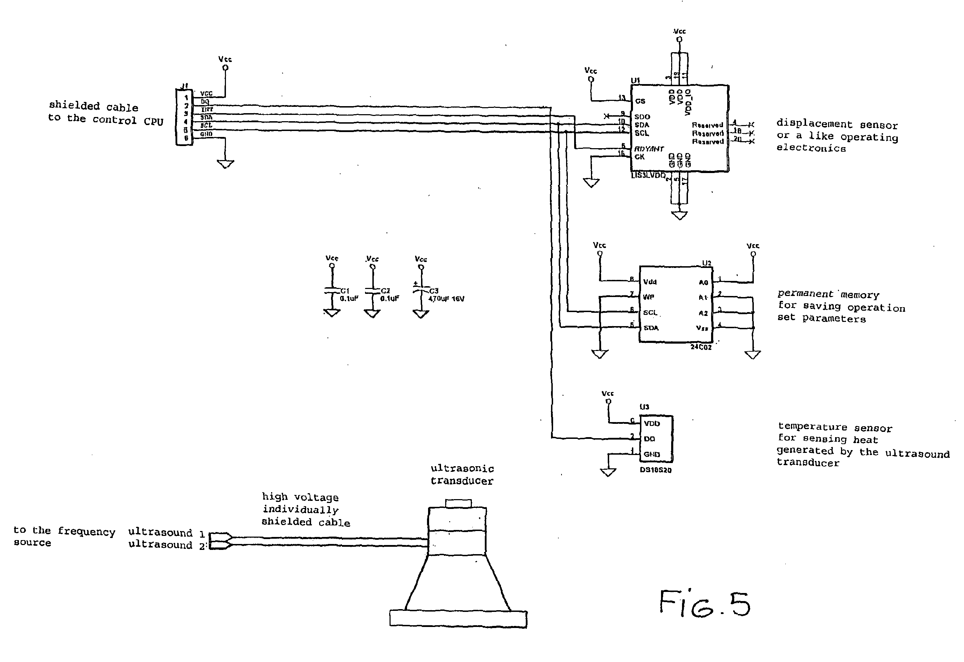

Figure 5 is an electric diagram of the ultrasound emitter.

### DESCRIPTION OF THE PREFERRED EMBODIMENTS

With reference to the number references of the above mentioned figures, the improved ultrasound device according to the present invention, which has been generally indicated by the reference number 1, comprises a device handle constituted by a box-like body which, in turn, preferably comprises two body half-shells, generally indicated by the reference numbers 2 and 3.

In the bottom half-shell 3, an ultrasound transducer 4, coupled to a vibrating part 5 provided for contacting a human body, is arranged.

Said vibrating part 5 advantageously comprises a contact plate, provided for contacting the user skin, and which oscillates at the same frequency as the oscillating frequency of the ultrasound transducer.

According to the present invention, the device comprises an electronic control system which, coupled to a CPU with a dedicated software, prevents the temperature of the device handle and related parts coupled thereto from increasing, thereby preventing the user skin surface from being burnt, and, moreover, being also designed for preventing the device handle from being held in a stationary condition at a single point, thereby preventing the tissue from being necrotized.

The subject device preferably operates in a frequency range from 22Khz to 150Khz, and being suitable to generate a cavitational effect inside the cellular tissues.

In addition to the above mentioned ultrasound transducer 4, the device according to the present invention further comprises an acceleration sensor 6 (or similar circuits adapted to provide the same function) allowing to detect any displacement of the ultrasound emitter, as well as a temperature sensor 7 applying on the skin contacting vibrating part 5.

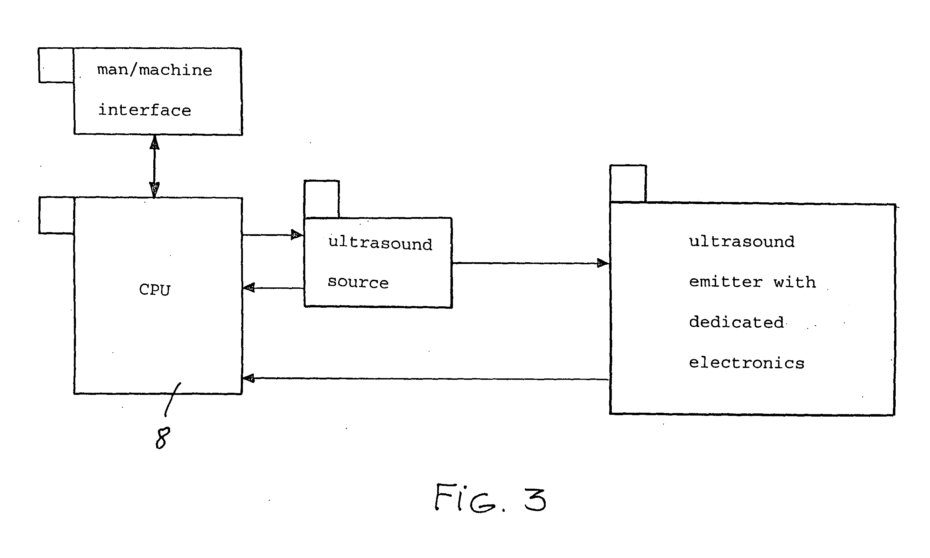

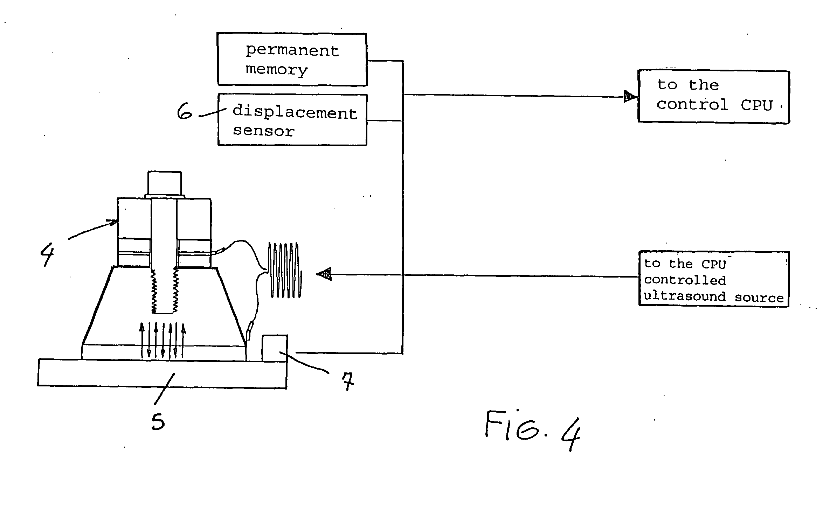

As is schematically shown in figures 3 and 4, said ultrasound emitter is coupled to the control apparatus.

Said control apparatus, as stated, is based on a CPU 8 and related dedicated running or operating software/firmware.

The software, in particular, operating said CPU 8, is adapted to detect, in cooperation with a related hardware, if the device handle is held for a time longer than a program preset time at a single position, for switching off and/or limit the ultrasound emission, to prevent any cellular damages from occurring.

Moreover, the apparatus is suitable to monitor the device handle temperature, and to switch off and/or limit the ultrasound emission and signal this to the operator, in a case said temperature would exceed present values, to prevent the user skin from being burnt and/or surface damaged.

The subject apparatus, furthermore, is designed for properly controlling and setting the ultrasound transducer oscillation frequency, to provide an optimum efficiency of said ultrasound transducer and/or to allow a user to properly choose a processing frequency mostly adapted for the treatment to be performed.

The above mentioned function can be carried out by said CPU by either exploiting or not technical data stored in a permanent memory 9, included in the ultrasound emitter electronic arrangement.

Said permanent memory will save any necessary ultrasound emitter data and/or further data necessary for a proper operation of the overall device.

In this connection it should be pointed out that said ultrasonic emitter will be connected to the control apparatus by conventional electric cables.

More specifically, said ultrasonic transducer is connected to the related frequency source arranged in the apparatus by two individually shielded cables, adapted to resist against high voltage, to prevent RF emissions through the encompassing environment, to reduce as far as possible undesired effects on the operator and person being subjected to the treatment.

The control apparatus will further comprise a man/machine interface, such as, for example, a display, an encoder, push button assemblies, Led's, indicating devices and so on.

It has been found that the invention fully achieves the intended aim and objects.

In fact, the invention provides an improved ultrasound device adapted to prevent the temperature of the device handle and parts coupled thereto from increasing, thereby preventing in turn the user skin from being surface burnt; moreover, the ultrasound emitter cannot be held stationary at a single point, thereby preventing the tissue from being necrotized.

In practicing the invention, the used materials, provided that they are compatible to the intended application, as well as the contingent size and shapes, can be any, according to requirements.

## Claims

1. An improved ultrasound device, specifically designed for treating fat or adipose tissue, **characterized in that** said device comprises a control apparatus which, being connected to a CPU with a dedicated software, prevents a temperature of a device handle and parts coupled thereto, from increasing so as to prevent a skin surface from being burnt, while preventing the ultrasound emitter from being held stationary at a single place, to prevent a tissue from being necrotized.

2. An improved ultrasound device, according to claim 1, **characterized in that** said device comprises a device handle including a box body comprising two box body bottom and top half-shells

3. An improved ultrasound device, according to claim 2, **characterized in that** said bottom half-shell comprises therein an ultrasound transducer coupled to a vibrating part contacting said body, said vibrating part comprising a vibrating part plate adapted to contact a user skin, said vibrating part oscillating with a same frequency as a frequency of said ultrasound transducer.

4. An improved ultrasound device, according to one or more of the preceding claims, **characterized in that** said ultrasound emitter operates with an operating frequency in a frequency range from 22Khz to 150Khz.

5. An improved ultrasound device, according to one or more of the preceding claims, **characterized in that** said device further comprises, in addition to said ultrasound transducer, an acceleration sensor or similar circuit performing a same function, allowing to detect a displacement of said ultrasound emitter, as well as a temperature sensor applied to the skin contacting part.

6. An improved ultrasound device, according to one or more of the preceding claims, **characterized in that** said ultrasound emitter is coupled to a control apparatus based on a CPU and dedicated operating software/firmware which, by said CPU and hardware, is adapted to detect if the device handle is held for a time longer than a set time at a single position, and being adapted to switch off and/or limit the ultrasound emissions, to prevent tissue cells from being damaged.

7. An improved ultrasound device, according to one or more of the preceding claims, **characterized in that** said control apparatus is further designed for monitoring the device handle temperature and switch off and/or limit the ultrasound emission and signal the operator if said ultrasound emission exceeds a preset value, to prevent the user skin from being burnt and/or surface damaged.

8. An improved ultrasound device, according to one or more of the preceding claims, **characterized in that** said control apparatus is so designed as to control and set the oscillation frequency of said ultrasound transducer to provide a maximum efficiency of said ultrasound transducer and/or to select the most suitable frequency for the treatment to be carried out; this function being performed by the CPU either exploiting or not technical data saved in a permanent memory included in the ultrasound emitter electronics.

9. An improved ultrasound device, according to one or more of the preceding claims, **characterized in that** said permanent memory is designed for saving data of said ultrasound emitter and/or further operating data.

10. An improved ultrasound device, according to one or more of the preceding claims, **characterized in that** said ultrasound transducer is coupled to a related frequency source, arranged in said control apparatus, by two individually shielded cables for a high voltage application, to prevent RF emissions to the encompassing environment and to reduce as far as possible undesired effects on an operator and the user being treated.

11. An improved ultrasound device, according to one or more of the preceding claims, **characterized in that** said control apparatus further comprises a man/machine interface such as a display, an encoder, push buttons, Led's, indicator devices and the like.
